# EUROPEAN PATENT APPLICATION

(11) **EP 3 884 928 A2**
(43) Date of publication of application: **29.09.2021**
(21) Application number: 19886937.2
(22) Date of filing: 20.11.2019
(51) Int. Cl.: A61K 8/368, A61Q 19/00, A61K 31/192, A61P 17/00

(54) **TOPICAL SKIN COMPOSITION COMPRISING DEHYDROABIETIC ACID FOR STRENGTHENING SKIN BARRIER OR MOISTURIZING SKIN**

(30) Priority: 21.11.2018 KR 20180144467
(71) Applicant: Amorepacific Corporation, Seoul 04386 (KR)
(72) Inventor: KANG, Young Gyu, Yongin-Si, Gyeonggi-do 17074 (KR); LEE, Eun Soo, Yongin-Si, Gyeonggi-do 17074 (KR); KIM, Dong Hyun, Yongin-Si, Gyeonggi-do 17074 (KR)
(74) Representative: Germain Maureau
(86) International application number: PCT/KR2019/015926
(87) International publication number: WO 2020/106055

(57) **Abstract**

The present invention relates to a topical skin composition comprising dehydroabietic acid, an isomer thereof, a precursor thereof, a salt thereof, a hydrate thereof, or a solvate thereof as an active ingredient for strengthening a skin barrier or moisturizing the skin. More specifically, the present invention relates to a topical skin composition that comprises dehydroabietic acid, an isomer thereof, a precursor thereof, a salt thereof, a hydrate thereof, or a solvate thereof to activate peroxisome proliferator-activated receptor alpha (PPAR α) in vivo to upregulate the expression thereof as well as the expression of filaggrin gene, transglutaminase 1 gene, and hyaluronic acid synthase 2 gene, whereby the composition induces the differentiation of skin keratinocytes, strengthens the skin barrier, and increases a skin water content.

## Description

### [Technical Field]

### Cross-reference to Related Application

This application claims the priority of Korean Patent Application No. 10-2018-0144467, filed on November 21, 2018, the contents of which in their entirety are herein incorporated by reference.

### Technical Field

The present disclosure relates to a skin external application composition for strengthening skin barrier or moisturizing skin, which contains dehydroabietic acid, an isomer thereof, a precursor thereof, a salt thereof, a hydrate thereof or a solvate thereof as an active ingredient.

### [Background Art]

Skin is largely divided into three layers of the epidermis, the dermis and the subcutaneous tissue sequentially from outside. It protects the organs of the body from change in temperature and humidity, UV, and other physical and chemical stimuli from external environment. In particular, the epidermis plays an important role in preventing the evaporation of water inside the human body.

The epidermis is subdivided into the stratum corneum, the stratum granulosum, the stratum spinosum and the stratum basale sequentially from outside. The cells of the stratum corneum function like bricks while the lipids between the keratinocytes function like mortar, thereby constituting a skin barrier *(*J. Invest. Dermatol. 80 (Suppl.), 44-49. 1983). In the keratinocytes of a healthy person, natural moisturizing factors (NMFs) are present in high concentrations and help to retain water. For example, water-soluble substances such as amino acids prevent drying of water in skin by effectively binding with water *(*J. Invest. Dermatol. 54, 24-31, 1970). However, the necessity for a composition for strengthening skin barrier or moisturizing skin is increasing because of the phenomena of dryness, roughening, crumbliness desiccatedness, etc. of skin caused by various reasons such as artificial temperature control for air conditioning due to change in environment or lifestyles, skin stress caused by various stresses from social lives and environmental pollution, frequent face washing due to increased makeup, natural skin aging, etc. Peroxisome proliferator-activated receptors (PPARs) are representative nuclear hormone receptors which play important roles in regulation of cellular proliferation/differentiation, regulation of inflammatory mediators, etc. and are reported to play important roles also in regulation of glucose, lipid and hormone metabolism (Kuenzli, S., Saurat, J. H., Peroxisome proliferator-activated receptors in cutaneous biology, Br. J. Dermatol., 149 (2003), 229-236; Desvergne, B., Wahli, W., Peroxisome proliferator-activated receptors: nuclear control of metabolism, Endocrine Reviews, 20 (1999) 649-688.). Especially, they have been found out to play important roles in maintaining the homoeostasis of skin functions by promoting keratinocyte differentiation and inhibiting keratinocyte proliferation in the epidermis, facilitating skin barrier formation through lipid metabolism, and inhibiting inflammatory responses.

### [Disclosure]

### [Technical Problem]

The inventors of the present disclosure have researched on the effect of plant-derived natural substances on skin. The present disclosure is directed to providing a composition which induces the differentiation of keratinocytes and strengthens skin barrier by activating peroxisome proliferator-activated receptor α (PPARα) and regulating the gene expression thereof using dehydroabietic acid which has been known to exhibit antiaging or skin-whitening effect.

### [Technical Solution]

The present disclosure provides a skin external application composition containing dehydroabietic acid, an isomer thereof, a precursor thereof, a salt thereof, a hydrate thereof or a solvate thereof as an active ingredient. The composition exhibits skin-moisturizing, keratinocyte-differentiating or skin barrier-strengthening effect.

In an exemplary embodiment, the composition may contain dehydroabietic acid, an isomer thereof, a precursor thereof, a salt thereof, a hydrate thereof or a solvate thereof in an amount of 0.001-10 wt% based on the total weight of the composition.

In an exemplary embodiment, the composition may induce the transcriptional activity of peroxisome proliferator-activated receptor α (PPARα), or may increase the expression of the filaggrin gene, the transglutaminase 1 (TGase 1) gene or the hyaluronic acid synthase 2 (HAS 2) gene.

In an exemplary embodiment, the composition may be a cosmetic composition.

In an exemplary embodiment, the composition may be a pharmaceutical composition.

### [Advantageous Effects]

The inventors of the present disclosure have identified that dehydroabietic acid induces the differentiation of keratinocytes and strengthens skin barrier by activating peroxisome proliferator-activated receptor α (PPARα) and regulating the gene expression thereof, and also increases the expression of the filaggrin gene which is a precursor protein of a natural moisturizing factor that is responsible for skin moisturization, the transglutaminase 1 gene which plays a critical role in the formation of cornified envelope, and the hyaluronic acid synthase 2 gene which is responsible for the synthesis of hyaluronic acid that is a component of the extracellular matrix which maintains water in skin, and have completed the present disclosure.

### [Brief Description of Drawings]

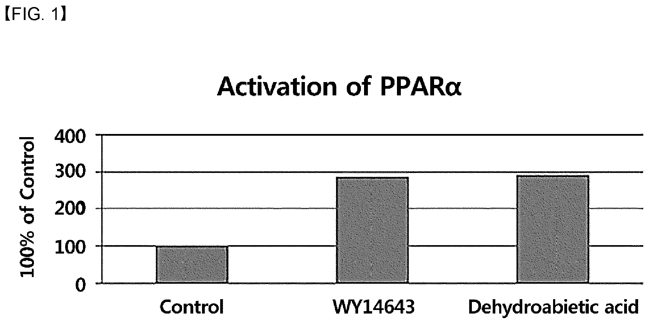

FIG. 1 shows the activation of PPARα in a negative control group wherein human keratinocytes are treated with ethanol, a positive control group wherein the cells are treated with WY14643, and a group wherein the cells are treated with dehydroabietic acid.

### [Best Mode]

The present disclosure provides a skin external application composition, which contains dehydroabietic acid, an isomer thereof, a precursor thereof, a salt thereof, a hydrate thereof or a solvate thereof as an active ingredient.

The "isomer" includes particularly an optical isomer, a conformational isomer, a position isomer (especially, a tautomer) or a geometric isomer.

The "precursor" refers to a compound which has been chemically modified to change physical and chemical properties, which does not exhibit physiological activity on its own but can exert a desired effect when converted to the original compound after being administered into the body by the action of an enzyme.

The "salt" refers to a "pharmaceutically acceptable salt", and the term "pharmaceutically acceptable" means generally being safe and nontoxic or biologically or otherwise useful in preparing a pharmaceutical composition, for not only veterinary but also pharmaceutical use in human.

The term "pharmaceutically acceptable salt" refers to a salt which is pharmaceutically acceptable as defined above and has a desired pharmacological activity. The salt includes: an acid addition salt which is formed from an inorganic salt, e.g., hydrochloric acid, hydrobromic acid, sulfuric acid, nitric acid, phosphoric acid, etc., or an organic acid, e.g., acetic acid, propionic acid, hexanoic acid, cyclopentylpropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxy ethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, 3-butylacetic acid, lauryl sulfuric acid, gluconic acid, glutamic acid, hydroxynaphthoic acid, salicylic acid, stearic acid, muconic acid, etc.; or a salt which is formed from as an acidic proton present in a parent compound is replaced.

The "hydrate" refers to a "pharmaceutically acceptable hydrate". The "hydrate" is formed when the compound of the present disclosure contains water. The hydrate may contain one or more water molecule per molecule of the compound of the present disclosure. Non-limiting examples include a monohydrate, a dihydrate, a trihydrate and a tetrahydrate. The hydrate may also contain one or more molecule of the compound of the present disclosure per water molecule. Non-limiting examples include a semihydrate. In an exemplary embodiment, water may be retained in crystals in various ways. Thus, water molecules may occupy lattice sites in the crystals, or may form bonding with a salt of the compound. The hydrate should be "acceptable", which means that it should not be harmful to the recipient.

The "solvate" refers to a "pharmaceutically acceptable solvate". The "solvate" means that the compound of the present disclosure contains one or more pharmaceutically acceptable solvent. The solvate may contain one or more solvent molecule per molecule of the compound of the present disclosure, or may contain one or more molecule of the compound of the present disclosure per solvent molecule. In an exemplary embodiment, the solvent may be retained in crystals in various ways. Thus, the solvent molecules may occupy lattice sites in the crystals, or may form bonding with a salt of the compound.

In an exemplary embodiment, the composition may be a skin external application composition for moisturizing skin, differentiating keratinocytes, or strengthening skin barrier.

In another aspect, the present disclosure may relate to a method for moisturizing skin, which comprises administering an effective amount of dehydroabietic acid, an isomer thereof, a precursor thereof, a salt thereof, a hydrate thereof or a solvate thereof to a subject in need thereof.

In another aspect, the present disclosure may relate to a use of dehydroabietic acid, an isomer thereof, a precursor thereof, a salt thereof, a hydrate thereof or a solvate thereof for preparing a composition for moisturizing skin.

In another aspect, the present disclosure may relate to dehydroabietic acid, an isomer thereof, a precursor thereof, a salt thereof, a hydrate thereof or a solvate thereof for use in moisturizing skin.

In another aspect, the present disclosure may relate to a use of dehydroabietic acid, an isomer thereof, a precursor thereof, a salt thereof, a hydrate thereof or a solvate thereof for moisturizing skin.

In another aspect, the present disclosure may relate to a method for differentiating keratinocytes, which comprises administering an effective amount of dehydroabietic acid, an isomer thereof, a precursor thereof, a salt thereof, a hydrate thereof or a solvate thereof to a subject in need thereof.

In another aspect, the present disclosure may relate to a use of dehydroabietic acid, an isomer thereof, a precursor thereof, a salt thereof, a hydrate thereof or a solvate thereof for preparing a composition for differentiating keratinocytes.

In another aspect, the present disclosure may relate to dehydroabietic acid, an isomer thereof, a precursor thereof, a salt thereof, a hydrate thereof or a solvate thereof for use in differentiating keratinocytes.

In another aspect, the present disclosure may relate to a use of dehydroabietic acid, an isomer thereof, a precursor thereof, a salt thereof, a hydrate thereof or a solvate thereof for differentiating keratinocytes.

In another aspect, the present disclosure may relate to a method for strengthening skin barrier, which comprises administering an effective amount of dehydroabietic acid, an isomer thereof, a precursor thereof, a salt thereof, a hydrate thereof or a solvate thereof to a subject in need thereof.

In another aspect, the present disclosure may relate to a use of dehydroabietic acid, an isomer thereof, a precursor thereof, a salt thereof, a hydrate thereof or a solvate thereof for preparing a composition for strengthening skin barrier.

In another aspect, the present disclosure may relate to dehydroabietic acid, an isomer thereof, a precursor thereof, a salt thereof, a hydrate thereof or a solvate thereof for use in strengthening skin barrier.

In another aspect, the present disclosure may relate to a use of dehydroabietic acid, an isomer thereof, a precursor thereof, a salt thereof, a hydrate thereof or a solvate thereof for strengthening skin barrier.

In an aspect of the present disclosure, the administration may be according to the administration method and administration dosage described in the present disclosure.

In an exemplary embodiment, the composition may be a skin external application composition for inducing the transcriptional activity of peroxisome proliferator-activated receptor α (PPARα), increasing the expression of the filaggrin gene, increasing the expression of the transglutaminase 1 (TGase 1) gene, or increasing the expression of the hyaluronic acid synthase 2 (HAS 2) gene.

In an exemplary embodiment, the content of the dehydroabietic acid, an isomer thereof, a precursor thereof, a salt thereof, a hydrate thereof or a solvate thereof may be 0.001-10 wt% based on the total weight of the composition. When the content is less than 0.001 wt%, the effect of inducing the differentiation of the epidermis, moisturizing skin and strengthening skin barrier may not be achieved. And, a content exceeding 10 wt% is inefficient because the increase in effect is not so much as the increase in the content.

In another aspect, the content of dehydroabietic acid may be 0.001 wt% or more, 0.01 wt% or more, 0.1 wt% or more, 0.5 wt% or more, 1 wt% or more, 1.5 wt% or more, 2 wt% or more, 2.5 wt% or more, 2.75 wt% or more, 3 wt% or more, 3.25 wt% or more, 3.5 wt% or more, 3.75 wt% or more, 4 wt% or more, 4.25 wt% or more, 4.5 wt% or more or 4.75 wt% or more, and 10 wt% or less, 9 wt% or less, 8.5 wt% or less, 8 wt% or less, 7.75 wt% or less, 7.5 wt% or less, 7.25 wt% or less, 7 wt% or less, 6.75 wt% or less, 6.5 wt% or less, 6.25 wt% or less, 6 wt% or less, 5.75 wt% or less, 5.5 wt% or less or 5.25 wt% or less, based on the total weight of the composition.

In an exemplary embodiment, the skin external application composition may induce the transcriptional activity of peroxisome proliferator-activated receptor α (PPARα). The increased activity of peroxisome proliferator-activated receptor α strengthens skin barrier and increases skin moisturization by inducing keratinocyte differentiation.

In an exemplary embodiment, the skin external application composition may increase the expression of the filaggrin gene. The filaggrin gene is a gene which expresses the precursor proteins of natural moisturizing factors (NMFs). The increased expression of the filaggrin gene increases skin moisturization.

In an exemplary embodiment, the skin external application composition may increase the expression of the transglutaminase 1 (TGase 1) gene. The transglutaminase 1 gene induces keratinocyte differentiation by forming cornified envelope.

In an exemplary embodiment, the skin external application composition may increase the expression of the hyaluronic acid synthase 2 (HAS 2) gene. The hyaluronic acid synthase 2 gene is responsible for the synthesis of hyaluronic acid. The increased expression of the gene improves the function of maintaining skin moisturization.

In an exemplary embodiment, the skin external application composition may be a cosmetic composition.

A formulation of the cosmetic composition is not particularly limited but may be selected adequately depending on purposes. For example, the cosmetic composition may be prepared into one or more formulation selected from a group consisting of a skin lotion, a skin softener, a skin toner, an astringent, a lotion, a milk lotion, a moisturizing lotion, a nourishing lotion, a massage cream, a nourishing cream, a moisturizing cream, a hand cream, a foundation, an essence, a nourishing essence, a pack, a soap, a cleansing foam, a cleansing lotion, a cleansing cream, a body lotion and a body cleanser, although not being limited thereto.

When the formulation of the present disclosure is a paste, a cream or a gel, animal fiber, plant fiber, wax, paraffin, starch, tragacanth, a cellulose derivative, polyethylene glycol, silicon, bentonite, silica, talc, zinc oxide, etc. may be used as a carrier ingredient.

When the formulation of the present disclosure is a powder or a spray, lactose, talc, silica, aluminum hydroxide, calcium silicate or polyamide powder may be used as a carrier ingredient. Particularly, a spray may additionally contain a propellant such as chlorofluorohydrocarbon, propane/butane or dimethyl ether.

When the formulation of the present disclosure is a solution or an emulsion, a solvent, solubilizer or an emulsifier may be used as a carrier ingredient. Examples include water, ethanol, isopropanol, ethyl carbonate, ethyl acetate, benzyl alcohol, benzyl benzoate, propylene glycol, 1,3-butylene glycol, aliphatic glycerol ester, polyethylene glycol or a fatty acid ester of sorbitan.

When the formulation of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier ingredient.

When the formulation of the present disclosure is a surfactant-containing cleanser, aliphatic alcohol sulfate, aliphatic alcohol ether sulfate, sulfosuccinic monoester, isethionate, an imidazolinium derivative, methyl taurate, sarcosinate, a fatty acid amide ether sulfate, an amidoalkyl betaine, an aliphatic alcohol, a fatty acid glyceride, a fatty acid diethanolamide, vegetable oil, a lanolin derivative, an ethoxylated glycerol fatty acid ester, etc. may be used as a carrier ingredient.

The cosmetic composition may further contain, in addition to the dehydroabietic acid, a functional additive and an ingredient added in a general cosmetic composition. The functional additive may include an ingredient selected from a group consisting of a water-soluble vitamin, an oil-soluble vitamin, a polypeptide, a polysaccharide, a sphingolipid and a seaweed extract.

In an exemplary embodiment, the skin external application composition may be a pharmaceutical composition.

The pharmaceutical composition may further contain a pharmaceutical adjuvant such as an antiseptic, a stabilizer, a wetting agent, an emulsification promoter, a salt and/or a buffer for controlling osmotic pressure, etc. and other therapeutically useful substances, and may be formulated into various forms for parenteral administration. It may be a formulation for transdermal administration, e.g., a lotion, an ointment, a gel, a cream, a patch or a spray, although not being limited thereto.

Determination of the administration dosage of the active ingredient is within the level of those skilled in the art. A daily administration dosage of the active ingredient varies depending on various factors such as the progression of a disease in a subject, age, health condition, complications, etc. In general, the composition may be administered at a dosage of 1 µg/kg to 200 mg/kg, specifically 50 µg/kg to 50 mg/kg, once to three times a day, for an adult. However, the administration dosage does not limit the scope of the present disclosure in any way.

Hereinafter, the present disclosure is described in more detail through examples. The examples are only for illustrating the present disclosure, and it will be obvious to those having ordinary knowledge in the art that the scope of the present disclosure is not limited by the examples.

The dehydroabietic acid of the present disclosure may be represented by Chemical Formula 1 (CAS No.: 1740-19-8).

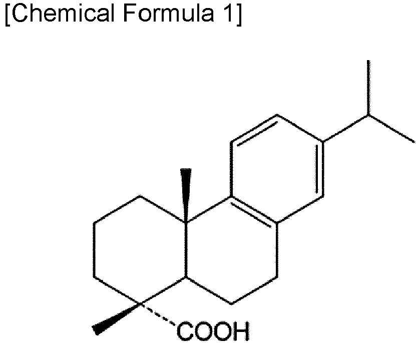

The dehydroabietic acid of the present disclosure induces the transcriptional activity of peroxisome proliferator-activated receptor α (PPARα) or induces keratinocyte differentiation by increasing expression thereof, thereby strengthening skin barrier and increasing skin moisturization. In addition, it provides a superior effect of inducing the differentiation of the epidermis, providing skin moisturization and strengthening skin barrier by increasing the expression of the filaggrin gene which is a precursor protein of a natural moisturizing factor (NMF) that is responsible for skin moisturization, the transglutaminase 1 (TGase 1) gene which plays a critical role in the formation of cornified envelope, and the hyaluronic acid synthase 2 (HAS 2) gene which is responsible for the synthesis of hyaluronic acid that is a component of the extracellular matrix which maintains water in skin.

### Example 1. Activation of peroxisome proliferator-activated receptor α (PPARα)

HaCaT cells, which are human keratinocytes, were subcultured in DMEM containing 10% fetal bovine serum. A phenol red-free medium was used to avoid the effect of phenol red on estrogen. The following plasmids were used: a plasmid having the PPARα gene downstream of a universal promoter which is expressed in general culture conditions; a plasmid having a PPAR response element (PPRE) activated by binding with ligand-bound PPAR as a promoter and a firefly luciferase gene reporter downstream of the promoter; and a reference plasmid having a β-galactosidase gene bound to a universal promoter [Kliewer, S. A., Forman, B. M., Blumberg, B., Ong, E. S., Borgmeyer, U., Mangelsdorf, D. J., Umesono, K., Evans, R. M., Differential expression and activation of a family of murine peroxisome proliferator-activated receptors, Proc. Natl. Acad. Sci. USA, 91 (1994) 7355-7359.].

The HaCaT cells were seeded onto a 24-well plate at a density of 5×10⁴ cells/well, cultured for 24 hours, and then transiently transfected with the plasmid genes. After 24 hours of culturing, followed by washing with phosphate-buffered saline (PBS), the ells were treated with dehydroabietic acid of Chemical Formula 1 purchased from Ramidus AB or WY14643, which is a PPARα ligand, as a positive control group at 10 µM. WY14643 is reported as a PPARα ligand which induces keratinocyte differentiation and strengthens skin barrier. A negative control group was treated with ethanol. After 24 hours of culturing, followed by washing with PBS, the cells were harvested and luciferase activity was measured. The result is shown in Table 1.

**[Table 1]**

| | PPARα activation (%) |
|---|---|
| Negative control group | 100 |
| WY14643 | 286.9 |
| Dehydroabietic acid | 291.5 |

Table 1 shows the result of transfecting the HaCaT cells with the PPRE promoter reporter plasmid and the PPARα expression plasmid, treating with test substances, and measuring luciferase activity. The result for each treatment group was represented relative to that of the negative control group. As seen from Table 1, dehydroabietic acid induced PPARα activation, since it exhibited luciferase activity comparable to that of the WY14643 positive control group (see FIG. 1).

### Example 2. Regulation of expression of PPARα. TGase 1. filaggrin and HAS2 genes

HaCaT cells were cultured in Dulbecco's modified Eagle's medium (DMEM, GIBCO 1210-0038) containing 10% fetal bovine serum. 1×10⁶ cells were placed in a 75T flask and cultured for 24 hours. Then, after treating with DMEM not containing fetal bovine serum and culturing for 24 hours, the cells were treated with dehydroabietic acid in a medium not containing fetal bovine serum for 24 hours. Then, after harvesting the cells and washing with cooled phosphate-buffered saline (PBS), total RNA was extracted by adding 1 mL of TRIzol™ reagent (Life Technologies, Inc.). Then, quantitative reverse transcriptase PCR was conducted. The primer sequences of PPARα, TGase 1, filaggrin and HAS 2 used to conduct PCR are described in Table 2. After adding 4 µg of total RNA to 25 µL of a reverse transcription buffer containing 50 mM Tris-HCI (pH 8.3), 75 mM KCI, 3 mM MgCl₂, 0.1 M DTT, 10 mM dNTP and 40 units/µL RNase inhibitor, 0.5 µg/µL oligo(dT)16 primer and 200 units of SuperScript II (GIBCO BRL) were added and incubation was conducted at 42 °C for 1 hour. Then, after mixing 2.5 µL of the reverse transcription solution with a 50 L of a PCR buffer containing AmpliTaq DNA polymerase (0.04U, Perkin Elmer, Shelton, CT), 50 mM Tris (pH 8.3), 0.25 mg/mL bovine serum albumin, 3 mM MgCl₂, 0.25 mM dNTPs and a 1/50,000 diluted solution of SYBR Green I (Molecular Probes, Eugene, OR), 10 µM of primers were added and PCR was conducted for 30 cycles of 30 seconds at 94 °C, 30 seconds at 53 °C and 1 minute at 72 °C. Relative mRNA level was analyzed by measuring the change in the fluorescence of SYBR Green I using the ICycler software. The result is shown in Table 3. WY14643, which is a PPARα ligand, was used as a positive control group. The relative mRNA level was represented relative to an ethanol-treated negative control group as 1.

**[Table 2]**

| Primer sequences used in PCR | | | | | | |
|---|---|---|---|---|---|---|
| NCBI Gene ID | Primer sequence | Sequence number | Amplification product size (bp) | PCR reaction temperature (°C) | | |
| | | | | Denaturation | Annealing | Extension |
| β-Actin | 5'-ATC CCA TCA CCA TCT TCC AG- 3' | 1 | 579 | 94 | 58 | 72 |
| | 5'-CCT GCT TCA CCA CCT TCT TG - 3' | 2 | | | | |
| PPARα | 5'-ATG TCC GAA GCA AAC ATC AC - 3' | 3 | 401 | 94 | 58 | 72 |
| | 5'-TAA TGT CCA GGA AGT AGG TG -3' | 4 | | | | |
| TGase1 | 5'-CCT GTA GCC CAC GTC GTA GC -3' | 5 | 374 | 94 | 58 | 72 |
| | 5'-TTG ACC TCA GCG CTG AGT TG -3' | 6 | | | | |
| Filaggrin | 5'-ACT CGG GGA TAA ATA GTT CCA A-3' | 7 | 357 | 94 | 60 | 70 |
| | 5'-CCT TAC ATA TAT ATT CCC CAG CAT-3' | 8 | | | | |
| HAS2 | 5'-TGA AGG ACC CTG CGA ACT | 9 | 185 | 95 | 61 | 70 |
| | TTC-3' | | | | | |
| | 5'-GAA CAC TCG CTC AAA CCA GC- 3' | 10 | | | | |
| | 5'-AGG TGT ATT GAC CCA TGC TAG AT-3' | 11 | | | | |

Table 3 shows the effect of the dehydroabietic acid according to the present disclosure on the mRNA expression level of PPARα, TGase1, filaggrin and HAS 2. The first column shows the result for an ethanol-treated negative control group, and the second column shows the result for a positive control group treated with WY14643 at different concentrations. WY14643 is a PPARα ligand reported to induce keratinocyte differentiation and strengthen skin barrier. As can be seen from Table 3, the treatment with dehydroabietic acid significantly increased the expression of the PPARα, transglutaminase 1 (TGase 1), filaggrin and hyaluronic acid synthase 2 (HAS 2) genes.

**[Table 3]**

| Gene expression level (fold) depending on concentration | | | | | |
|---|---|---|---|---|---|
| | Negative control | WY14643 | | Dehydroabietic acid | |
| | - | 1 µM | 10 µM | 1 µM | 10 µM |
| PPARα | 1 | 2.54 | 5.96 | 2.65 | 6.16 |
| TGase1 | 1 | 2.13 | 4.98 | 2.58 | 5.94 |
| Filaggrin | 1 | 1.86 | 3.12 | 2.31 | 3.34 |
| HAS 2 | 1 | 9.64 | 35.93 | 12.4 | 40.15 |

As described above, the dehydroabietic acid according to the present disclosure exhibited the effect of inducing keratinocyte differentiation, strengthening skin barrier and increasing skin moisturization by inducing the transcriptional activity of PPARα or increasing the expression thereof. In addition, it increased the expression of the filaggrin gene which is a precursor protein of a natural moisturizing factor (NMF) that is responsible for skin moisturization, the transglutaminase 1 (TGase 1) gene which plays a critical role in the formation of cornified envelope, and the hyaluronic acid synthase 2 (HAS 2) gene which is responsible for the synthesis of hyaluronic acid that is a component of the extracellular matrix which maintains water in skin.

Through this, the present disclosure can provide a skin external application composition for strengthening skin barrier or moisturizing skin, which contains dehydroabietic acid as an active ingredient.

Hereinafter, formulation examples of a composition containing the dehydroabietic acid of the present disclosure as an active ingredient are described. However, the composition of the present disclosure is not limited to the following examples.

### [Formulation Example 1] Lotion

**[Table 4]**

| Ingredients | Contents (wt%) |
|---|---|
| Dehydroabietic acid | 0.50 |
| Magnesium L-ascorbyl-2-phosphate | 1.00 |
| Water-soluble collagen (1% aqueous solution) | 1.00 |
| Sodium citrate | 0.10 |
| Citric acid | 0.05 |
| Licorice extract | 0.20 |
| 1,3-Butylene glycol | 3.00 |
| Purified water | Balance |

### [Formulation Example 2] Cream

**[Table 5]**

| Ingredients | Contents (wt%) |
|---|---|
| Dehydroabietic acid | 1.00 |
| Polyethylene glycol monostearate | 2.00 |
| Self-emulsifying glyceryl monostearate | 5.00 |
| Cetyl alcohol | 4.00 |
| Squalene | 4.00 |
| Glyceryl tri-2-ethylhexanoate | 6.00 |
| Sphingolipid | 1.00 |
| 1,3-Butylene glycol | 7.00 |
| Purified water | Balance |

### [Formulation Example 3] Pack

**[Table 6]**

| Ingredients | Contents (wt%) |
|---|---|
| Dehydroabietic acid | 3.00 |
| Polyvinyl alcohol | 13.00 |
| Magnesium L-ascorbyl-2-phosphate | 1.00 |
| Lauroyl hydroxyproline | 1.00 |
| Water-soluble collagen (1% aqueous solution) | 2.00 |
| 1,3-Butylene glycol | 3.00 |
| Ethanol | 5.00 |
| Purified water | Balance |

### [Formulation Example 4] Beauty solution

**[Table 7]**

| Ingredients | Contents (wt%) |
|---|---|
| Dehydroabietic acid | 0.10 |
| Hydroxyethyl cellulose (3% aqueous solution) | 12.00 |
| Xanthan gum (2% aqueous solution) | 2.00 |
| 1,3-Butylene glycol | 6.00 |
| Thick glycerin | 4.00 |
| Sodium hyaluronate (1% aqueous solution) | 5.00 |
| Purified water | Balance |

### [Sequence List Free-text]

SEQ ID NO 1
   atcccatcac catcttccag 20
SEQ ID NO 2
   cctgcttcac caccttcttg 20
SEQ ID NO 3
   atgtccgaag caaacatcac 20
SEQ ID NO 4
   taatgtccag gaagtaggtg 20
SEQ ID NO 5
   cctgtagccc acgtcgtagc 20
SEQ ID NO 6
   ttgacctcag cgctgagttg 20
SEQ ID NO 7
   actcggggat aaatagttcc aa 22
SEQ ID NO 8
   ccttacatat atattcccca gcat 24
SEQ ID NO 9
   tgaaggaccc tgcgaacttt c 21
SEQ ID NO 10
   gaacactcgc tcaaaccagc 20
SEQ ID NO 11
   aggtgtattg acccatgcta gat 23

<110>    AMOREPACIFIC CORPORATION

<120>    COMPOSITION FOR SKIN BARRIER STRENGTHENING OR MOISTURIZING
         EXTERNAL SKIN APPLICATION CONTAINING DEHYDROGENATED ABIETIC ACID

<130>    18P498IND

<160>    11

<170>    KoPatentIn 3.0

<210>    1
<211>    20
<212>    DNA
<213>    Artificial Sequence

<220>
<223>    Primer of beta-actin


<400>    1
atcccatcac catcttccag                                                   20


<210>    2
<211>    20
<212>    DNA
<213>    Artificial Sequence

<220>
<223>    Primer of beta-actin


<400>    2
cctgcttcac caccttcttg                                                   20


<210>    3
<211>    20
<212>    DNA
<213>    Artificial Sequence

<220>
<223>    Primer of PPARalpha


<400>    3
atgtccgaag caaacatcac                                                   20


<210>    4
<211>    20
<212>    DNA
<213>    Artificial Sequence

<220>
<223>    Primer of PPARalpha


<400>    4
taatgtccag gaagtaggtg                                                   20

<210>    5
<211>    20
<212>    DNA
<213>    Artificial Sequence

<220>
<223>    Primer of TGase1


<400>    5
cctgtagccc acgtcgtagc                                                    20


<210>    6
<211>    20
<212>    DNA
<213>    Artificial Sequence

<220>
<223>    Primer of TGase1


<400>    6
ttgacctcag cgctgagttg                                                    20


<210>    7
<211>    22
<212>    DNA
<213>    Artificial Sequence

<220>
<223>    Primer of Filaggrin


<400>    7
actcggggat aaatagttcc aa                                                 22


<210>    8
<211>    24
<212>    DNA
<213>    Artificial Sequence

<220>
<223>    Primer of Filaggrin


<400>    8
ccttacatat atattcccca gcat                                               24


<210>    9
<211>    21
<212>    DNA
<213>    Artificial Sequence

<220>
<223>    Primer of HAS2


<400>    9
tgaaggaccc tgcgaacttt c                                                  21

<210> 10
<211> 20
<212> DNA
<213> Artificial Sequence

<220>
<223> Primer of HAS2


<400> 10
gaacactcgc tcaaaccagc 20


<210> 11
<211> 23
<212> DNA
<213> Artificial Sequence

<220>
<223> Primer of HAS2


<400> 11
aggtgtattg acccatgcta gat 23

## Claims

1. A skin external application composition for moisturizing skin, comprising dehydroabietic acid, an isomer thereof, a precursor thereof, a salt thereof, a hydrate thereof or a solvate thereof as an active ingredient.

2. A skin external application composition for differentiating keratinocytes, comprising dehydroabietic acid, an isomer thereof, a precursor thereof, a salt thereof, a hydrate thereof or a solvate thereof as an active ingredient.

3. A skin external application composition for strengthening skin barrier, comprising dehydroabietic acid, an isomer thereof, a precursor thereof, a salt thereof, a hydrate thereof or a solvate thereof as an active ingredient.

4. The skin external application composition according to claim 3, wherein the composition strengthens skin barrier by inducing the transcriptional activity of peroxisome proliferator-activated receptor α (PPARα).

5. The skin external application composition according to claim 1, wherein the composition enhances skin moisturization by increasing the expression of the filaggrin gene.

6. The skin external application composition according to claim 2, wherein the composition differentiates keratinocytes by increasing the expression of the transglutaminase 1 (TGase 1) gene.

7. The skin external application composition according to claim 1, wherein the composition enhances skin moisturization by increasing the expression of the hyaluronic acid synthase 2 (HAS 2) gene.

8. The skin external application composition according to any of claims 1 to 3, wherein the dehydroabietic acid, an isomer thereof, a precursor thereof, a salt thereof, a hydrate thereof or a solvate thereof is comprised with a content of 0.001-10 wt% based on the total weight of the composition.

9. The skin external application composition according to any of claims 1 to 3, wherein the composition is a cosmetic composition.

10. The skin external application composition according to any of claims 1 to 3, wherein the composition is a pharmaceutical composition.
